# EUROPEAN PATENT APPLICATION

(11) **EP 4 011 410 A1**
(43) Date of publication of application: **15.06.2022**
(21) Application number: 21200461.8
(22) Date of filing: 01.10.2021
(51) Int. Cl.: A61L 27/16, A61L 27/56

(54) **SCAFFOLD FOR ARTIFICIAL ORGAN USING ACRYLIC SYNTHETIC POLYMER AND PREPARATION METHOD THEREOF**

(30) Priority: 11.12.2020 KR 20200173646
(71) Applicant: TE Bios Co., Ltd., Chungcheongbuk-do (KR)
(72) Inventor: KIM, Byeong Kook, 28160 Chungcheongbuk-do (KR); KIM, Tae Hyun, 31091 Chungcheongnam-do (KR); JEONG, Do Sun, 28162 Chungcheongbuk-do (KR)
(74) Representative: Yip, Matthew Wing Yu

(57) **Abstract**

The present invention relates to a scaffold for artificial organs using a watersoluble non-degradable acrylic polymer and a water-insoluble non-degradable acrylic polymer, which are acrylic synthetic polymers, and a preparation method thereof.

The acrylic monomer or polymer-based scaffold of the present invention improves the disadvantages of the natural polymer-based scaffolds, allowing degradation of specific sites *in vivo* and easy cell adhesion, and since it is possible to control mechanical properties, it is possible to implement mechanical properties and morphology suitable for where to use and purpose of use. In addition, the present invention can be applied as a biomaterial for artificial organs such as artificial cornea, artificial liver, artificial heart, artificial cartilage or artificial bone tissue, which requires maintenance of physical properties, high cell compatibility and stability in the body.

## Description

### CROSS-REFERENCE TO RELATED APPLICATION

This application claims priority to and the benefit of Korean Patent Application No. 10-2020-0173646, filed on Dec. 11, 2020, the disclosure of which is incorporated herein by reference in its entirety.

### [Technical Field]

The present invention relates to a scaffold for artificial organs using a water-soluble non-degradable acrylic polymer and a water-insoluble non-degradable acrylic polymer, which are acrylic synthetic polymers, and a preparation method thereof.

### [Background Art]

Regenerative medicine is a medical field that replaces or restores human cells, tissues or organs such that they can perform their original functions in addition to restoring their shape, when human cells, tissues or organs are damaged by accidents, aging or disease, and it is a field that studies a wide range of diseases, from simple fractures and skin injuries to incurable diseases for which there is no treatment, such as dementia, spinal cord injury, diabetes and the like.

Tissue engineering, which is a field of regenerative medicine, is being studied for various organs of the human body, such as bones, cartilage, blood vessels and the like, and various biomaterials are used as materials to restore, maintain and repair damaged tissues or organs. Therefore, it is important that biomaterials used for tissue engineering have high biocompatibility and the ability to implement mechanical properties and morphological properties suitable for function.

Scaffolds refer to structures that serve as a support such that tissue cells can make a three-dimensional living tissue. Scaffolds are in the spotlight as a basic technology that can implement artificial organs, and research on scaffolds for various organs is being conducted.

For most of the scaffolds being researched for tissue engineering, naturally derived polymers such as collagen, alginate or the like are used. Natural polymers have the advantages of non-toxicity and excellent biocompatibility, but since they are polysaccharide-based polymers containing unstable glycosidic bonds, when a biodegradable scaffold made of natural polymer materials is transplanted, it has disadvantages in that the degradation rate in the body is fast, the mechanical properties are weak, and it is degraded before the tissue is sufficiently regenerated by binding to the cells, and thus, it is not suitable to be utilized as a scaffold for artificial organs with a complex structure or to function continuously in the body.

In order to overcome the above disadvantages, methods such as increasing physical strength by using mixed polymers of natural and synthetic, increasing stability by synthesizing new functional groups or the like have been extensively studied, and techniques for forming pores in scaffolds to improve biocompatibility to be adhered to the surface of scaffolds and the adhesion, mobility and proliferation of cells are also being developed for tissue engineering applications.

Currently, although there is the method of salt leaching, salt foaming, highpressure gas expansion, emulsion freeze-drying, phase separation and etc. for forming pore in scaffold, the above methods may give unexpected deformation to the scaffolds by using high pressure and high temperature and have disadvantages of poor reproducibility.

### [Related Art Documents]

### [Patent Documents]

(Patent Document 1) KR 10-2015-0049291 A (May 8, 2015)

### [Disclosure]

### [Technical Problem]

As such, as a result of efforts to provide a porous biomaterial using a non-degradable acrylic polymer and a preparation method thereof, the inventors of the present invention confirmed that by using an organic solvent extraction method using localization of ionized water and a polar organic solvent, it is possible to fabricate a scaffold capable of controlling the size or porosity, and thereby completed the present invention.

Accordingly, an object of the present invention is to provide a scaffold for artificial organs using a water-soluble non-degradable acrylic monomer or polymer and a water-insoluble non-degradable acrylic monomer or polymer, which are acrylic synthetic polymers, and a preparation method thereof.

### [Technical Solution]

The present invention provides a method for preparing a scaffold, including:
i) preparing a mixed solution by mixing a water-soluble non-degradable acrylic monomer and a water-insoluble non-degradable acrylic monomer with distilled water;
ii) adding a salt and a polar solvent to the mixed solution of step i) to stir; and
iii) cross-linking the water-soluble non-degradable acrylic monomer in the stirred mixed solution of step ii) for polymerizing.

According to a preferred exemplary embodiment of the present invention, the water-soluble non-degradable acrylic monomer of step i) is at least one selected from the group consisting of hydroxymethacrylate, hydroxyethylacrylate, ethylacrylate, alkylacrylate, arylacrylate and cyanoacrylate.

According to a preferred exemplary embodiment of the present invention, the water-insoluble non-degradable acrylic monomer of step i) is at least one selected from the group consisting of methylmethacrylate, methylacrylate, alkylmethacrylate, arylmethacrylate and cyanomethacrylate.

According to a preferred exemplary embodiment of the present invention, the water-soluble non-degradable acrylic monomer and the water-insoluble non-degradable acrylic monomer of step i) are mixed at a weight ratio of 50 to 80 : 20 to 50.

According to a preferred exemplary embodiment of the present invention, the cross-linking, for polymerization, is at least one selected from the group consisting of chemical cross-linking, physical cross-linking, ionic cross-linking and radiation cross-linking.

According to a preferred exemplary embodiment of the present invention, an initiator and a catalyst for the chemical cross-linking, for polymerization, are included at 1 to 3 wt.% and 0.1 to 1 wt.% based on the total solution weight, respectively.

According to a preferred exemplary embodiment of the present invention, the initiator and the catalyst are included at a weight ratio of 50 to 80 : 20 to 50.

According to a preferred exemplary embodiment of the present invention, the radiation dose for the radiation cross-linking, for polymerization, is 10 kGy to 200 kGy.

According to a preferred exemplary embodiment of the present invention, the radiation is at least one selected from the group consisting of gamma rays, electron beam, ion beam and neutron beam.

According to a preferred exemplary embodiment of the present invention, the scaffold is in the form of a bead or sponge.

According to a preferred exemplary embodiment of the present invention, the scaffold is a scaffold for artificial organs.

In addition, the present invention provides a scaffold prepared by the above method.

### [Advantageous Effects]

The acrylic monomer or polymer-based scaffold of the present invention improves the disadvantages of the natural polymer-based scaffolds, allowing degradation of specific sites *in vivo* and easy cell adhesion, and since it is possible to control mechanical properties, it is possible to implement mechanical properties and morphology suitable for where to use and purpose of use. In addition, the present invention can be applied as a biomaterial for artificial organs such as artificial cornea, artificial liver, artificial heart, artificial cartilage or artificial bone tissue, which requires maintenance of physical properties, high cell compatibility and stability in the body.

### [Description of Drawings]

FIG. 1 is a set of images showing various types of scaffolds of the present invention.
FIG. 2 is a set of scanning electron microscope images showing two porous structures (bead and sponge) according to various composition ratios of the present invention.
FIG. 3 is a set of scanning electron microscope images showing changes in the number and size of porosity according to various composition ratios of the present invention.
FIG. 4 is a set of scanning electron microscope images showing the cell adhesion of two porous structure (bead and sponge) scaffolds.
FIG. 5 is a set of histological images showing the biocompatibility of bead-type scaffolds by a rat subcutaneous implantation test.
FIG. 6 is a set of histological images showing the biocompatibility of sponge-type scaffolds by a rat subcutaneous implantation test.

### [Modes of the Invention]

Hereinafter, the present invention will be described in more detail.

Biomaterials for use in tissue engineering should sufficiently perform the role of a framework through a three-dimensional structure such that surrounding tissue cells may adhere to the surface of the material and become organized for the regeneration of human tissue, and after implantation, it should have biocompatibility such that blood clotting or inflammation does not occur or is minimal. An ideal biocompatible material needs mechanical properties suitable for the specific organ *in vitro* or *in vivo,* the ability to induce cell infiltration, the ability to help the growth and differentiation of cells or the ability to supply oxygen or nutrients to maintain cell homeostasis.

A porous polymer material is suitable to satisfy the above essential requirements because it is possible to control cell compatibility or biocompatibility through porosity control and induce physical properties suitable for the specific organ through the control of mechanical properties.

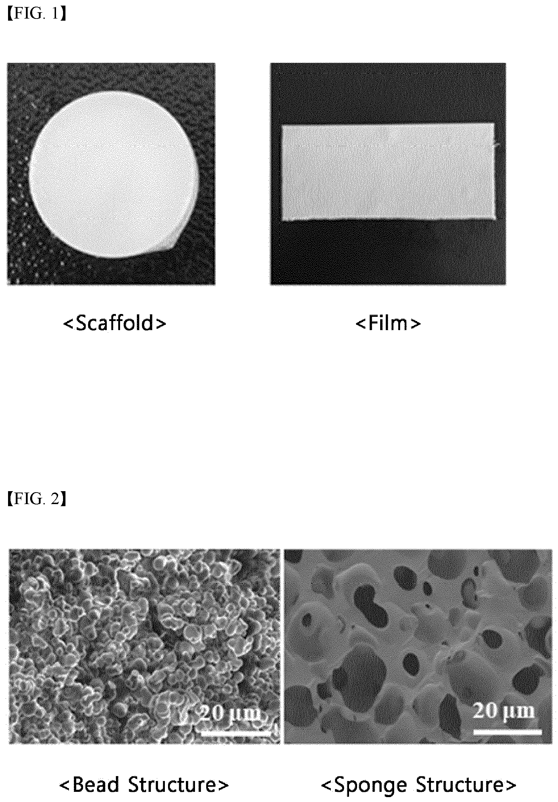

As such, by using a method of made a polymer by monomers or directly using a polymer to mix and cross-link, the present invention is directed to providing a scaffold that exhibits desired physical properties without a complex process by mixing a water-soluble non-degradable acrylic polymer and a water-insoluble non-degradable acrylic polymer having biocompatibility at an appropriate ratio. The scaffold according to the present invention may be prepared in various forms, such as a film, a scaffold or the like, depending on the shape of a mold (FIG. 1).

Accordingly, the present invention provides a method for preparing a scaffold, including:
i) preparing a mixed solution by mixing a water-soluble non-degradable acrylic monomer and a water-insoluble non-degradable acrylic monomer with distilled water;
ii) adding a salt and a polar solvent to the mixed solution of step i) to stir; and
iii) cross-linking the water-soluble non-degradable acrylic monomer in the stirred mixed solution of step ii) for polymerizing.

According to a preferred exemplary aspect of the present invention, the water-soluble non-degradable acrylic monomer of step i) may be at least one selected from the group consisting of hydroxymethacrylate, hydroxyethylacrylate, ethylacrylate, alkylacrylate, arylacrylate and cyanoacrylate. The water-soluble non-degradable acrylic monomer may be included in an amount of 10 to 30 wt.% based on the total weight. The water-soluble acrylic monomer has a hydrophilic property and may play a role in backbone of fabricated a scaffold.

According to a preferred exemplary aspect of the present invention, the water-insoluble non-degradable acrylic monomer of step i) may be at least one selected from the group consisting of methylmethacrylate, methylacrylate, alkylmethacrylate, arylmethacrylate and cyanomethacrylate. The water-insoluble non-degradable acrylic monomer may be included in an amount of 0.5 to 8 wt.% based on the total weight. The water-insoluble acrylic monomer exhibits hydrophobic properties and may serve to maintain mechanical strength.

According to a preferred exemplary aspect of the present invention, the water-soluble non-degradable acrylic monomer and the water-insoluble non-degradable acrylic monomer of step i) may be mixed at a weight ratio of 50 to 80 : 20 to 50. More preferably, the water-soluble non-degradable acrylic monomer and the water-insoluble non-degradable acrylic monomer may be mixed at a weight ratio of 60 to 80 : 20 to 40. When the water-insoluble acrylic monomer is added more than the water-soluble acrylic monomer, the scaffold may not be fabricated.

In addition, the present invention may induce pore formation by using a salt and a polar solvent to induce cell infiltration of the scaffold. In this process, the salt and the polar solvent may localized the solvent to induce pore formation.

The salt of step ii) may be at least one selected from the group consisting of crystalline salts such as sodium chloride (NaCl), potassium chloride (KCl), magnesium chloride (MgCl) and the like, crystalline hydroxides such as calcium hydroxide and the like, and water-soluble polysaccharides such as sugar, starch and the like. The weight ratio of the salt may be 0.05 to 0.5 wt.% based on the total weight.

The polar solvent in step ii) may be a polar organic solvent. Preferably, it may be at least one selected from the group consisting of dimethylformamide, dimethyl sulfoxide, acetone and acrylonitrile. In this case, the weight ratio of the polar solvent may be 5 to 20 wt.% based on the total weight.

According to a preferred exemplary aspect of the present invention, the cross-linking method, for polymerization, may be at least one selected from the group consisting of chemical cross-linking, physical cross-linking, ionic cross-linking and radiation cross-linking. In this case, the time may be 30 minutes to 10 hours.

For the chemical cross-linking, for polymerization, a chemical cross-linking agent may be used, or for the physical cross-linking, for polymerization, the freeze-thawing method may be used, and for the ionic cross-linking, for polymerization, a divalent or higher cation may be used. The radiation may be at least one selected from the group consisting of gamma rays, electron beam, ion beam and neutron beam, and preferably electron beam.

The chemical cross-linking agent may be an acrylic polymer including two or more carboxylic acids in monomers, and preferably, it may be pentaerythritol tetraacrylate (PETA) having four carboxylic acids. The chemical cross-linking agent may be included in an amount of 0.1 to 5 wt.%, and more preferably, 0.5 to 2 wt.%, based on the total weight.

According to a preferred exemplary aspect of the present invention, an initiator and a catalyst for the chemical cross-linking, for polymerization, may be included at 1 to 3 wt.% and 0.1 to 1 wt.% based on the total solution weight, respectively. The initiator and the catalyst may be ammonium persulfate (APS) and N,N,N',N'-tetramethylethylenediamine (TEMED), respectively.

According to a preferred exemplary aspect of the present invention, the initiator and the catalyst may be included at a weight ratio of 50 to 80 : 20 to 50. Preferably, the initiator and the catalyst may be included at a weight ratio of 60 to 80 : 20 to 40. If the catalyst is included more than the initiator, the catalyst may remain.

According to a preferred exemplary aspect of the present invention, the radiation dose for the radiation cross-linking, for polymerization, may be 10 kGy to 200 kGy, and preferably, the radiation dose may be 50 kGy to 100 kGy.

According to a preferred exemplary aspect of the present invention, the radiation may be at least one selected from the group consisting of gamma rays, electron beam, ion beam and neutron beam, and preferably, electron beam.

In the case of using a polymer, not a monomer, in an exemplary aspect of the present invention, a scaffold may be prepared using only steps ii) and iii). In this case, the water-soluble non-degradable acrylic polymer may be polyhydroxymethacrylate, polyhydroxyethylacrylate, polyethylacrylate, polyalkylacrylate, polyarylacrylate and polycyanoacrylate or a copolymer thereof, and the water-insoluble non-degradable acrylic polymer may be polymethylmethacrylate, polymethylacrylate, polyalkylmethacrylate, polyarylmethacrylate and polycyanomethacrylate or a copolymer thereof.

In addition, the present invention provides a scaffold prepared by the above method.

According to a preferred exemplary aspect of the present invention, the scaffold may be in the form of a bead or sponge.

According to a preferred exemplary aspect of the present invention, the scaffold may be a scaffold for artificial organs.

Hereinafter, examples will be provided to describe the present specification in detail. However, the examples according to the present specification may be modified in various other forms, and the scope of the present specification is not to be construed as being limited to the examples described below. The examples of the present specification are provided to describe the present specification more completely to those of ordinary skill in the art.

### [Example 1 ]

### Physical properties of scaffolds according to composition ratios

### <1-1> Preparation of scaffold

30 parts by weight of methyl methacrylate (MMA) and 70 parts by weight of 2-hydroxyethyl methacrylate (HEMA); 50 parts by weight of MMA and 50 parts by weight of HEMA; and 70 parts by weight of MMA and 30 parts by weight of HEMA were placed in distilled water to prepare a solution. In this case, the total contents of MMA and HEMA in the solution were fixed to 50 wt.%. Sodium chloride (NaCl), dimethylformamide (DMF) and pentaerythritol tetraacrylate (PETA) were added to the solution. In this case, NaCl was adjusted to 1 wt.% based on the total solution weight, DMF was adjusted to 5 wt.% based on the total solution weight, and PETA was adjusted to 1 wt.% with respect to HEMA. After the solution was stirred, ammonium persulfate (APS) and N,N,N',N'-tetramethylethylenediamine (TEMED) were added to cross-link and polymerize MMA and HEMA at room temperature. In this case, APS and TEMED were added in an amount of 1 wt.% based on the MMA and HEMA contents, respectively, to prepare a mixed solution.

Afterwards, mixed the solutions in the Eppendorf tube and inject to a Teflon mold (depth 0.5 mm, width 35 mm, length 28 mm). The solution in the mold were then polymerized for 1hour in the oven at 37°C, and the scaffold was prepared by separation from the mold.

### <1-2> Measurement of maximum tensile strength

In order to confirm the mechanical properties of the scaffold prepared in Example <1-1>, ultimate strength was measured. H5KT (Tinius Olsen Horizon) was used as the equipment, and the change in tensile strength was confirmed until the sample was crushed under the conditions of pre-load 0.02N and speed 5 mm/min.

### <1-3> Confirmation of average pore size and morphology

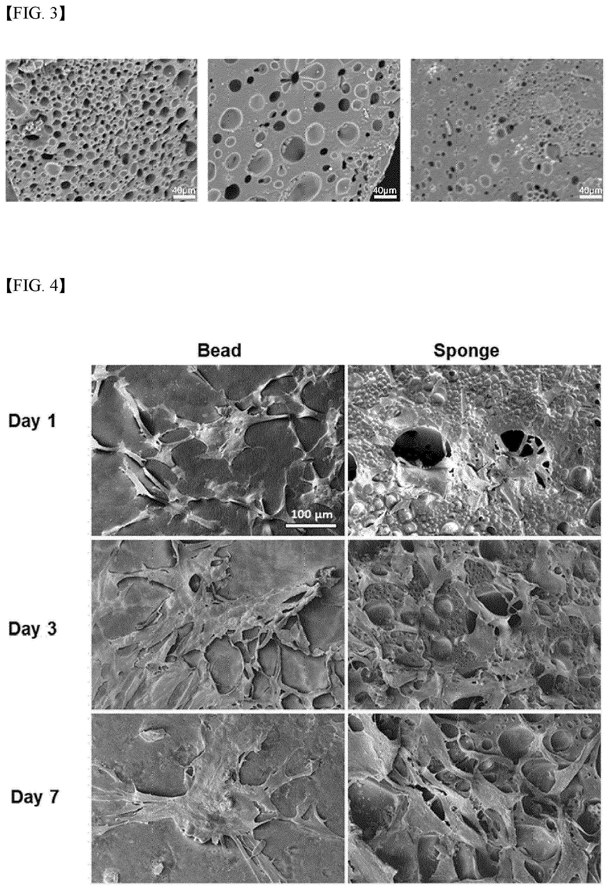

After the scaffold prepared in Example <1-1> was coated with platinum, the average pore size and the morphology of the scaffold were confirmed using a scanning electron microscope (SEM) (FIG. 2 and FIG. 3).

**[Table 1]**

| **MMA:HEMA composition ratio** | **Ultimate strength** | **Average pore size** | **Morphology** |
|---|---|---|---|
| 70:30 | 324±24kPa | 26±5µm | Bead |
| 50:50 | 314±35kPa | 28±4µm | Bead |
| 30:70 | 291±22kPa | 21±6µm | Bead |

As a result, as shown in [Table 1], it was confirmed that the ultimate strength increased as the content of MMA increased.

### [Example 2]

### Physical properties of scaffolds according to weight ratios

30 parts by weight of methyl methacrylate (MMA) and 70 parts by weight of 2-hydroxyethyl methacrylate (HEMA) were added to distilled water to prepare a solution. In this case, the distilled water was adjusted to 30 wt.%; 50 wt.%; or 70 wt.% based on the total solution weight, respectively.

Afterwards, scaffolds were prepared in the same manner as in <Example 1>, and the mechanical properties thereof were confirmed.

**[Table 2]**

| **Weight ratio of distilled water based on total solution weight** | **Ultimate strength** | **Average pore size** | **Morphology** |
|---|---|---|---|
| 30% | 315±41kPa | 54±12µm | Sponge |
| 50% | 294±42kPa | 26±4µm | Bead |
| 70% | 276±38kPa | 24±12µm | Bead |

As a result, as shown in [Table 2], as the content of distilled water increased, the ultimate strength decreased, and it was confirmed that the scaffold changed to a bead shape.

### [Example 3]

### Physical properties of scaffolds according to preparation methods

30 parts by weight of methyl methacrylate (MMA) and 70 parts by weight of 2-hydroxyethyl methacrylate (HEMA) were added to distilled water to prepare a solution. In this case, the total contents of MMA and HEMA in the solution were fixed to 50 wt.%. Sodium chloride (NaCl) and dimethylformamide (DMF) were added to the solution. In this case, NaCl was adjusted to 1 wt.% based on the total solution weight, and DMF was adjusted to 5 wt.% based on the total solution weight.

After filling the prepared solution into a Teflon mold, electron beam at a dose of 50 KGy or 100 KGy was irradiated by adjusting the energy, current, movement speed and number of irradiations. In this case, the dose was fixed at 50KGy under conditions in which the energy was 5 MeV, the current was 380 to 420 mA, and the moving speed was 1 m/min, and it was irradiated once or twice to set 50 KGy and 100 KGy conditions, respectively.

**[Table 3]**

| **Irradiation dose of electron beam** | **Ultimate strength** | **Average pore size** | **Morphology** |
|---|---|---|---|
| 50KGy | 242±24kPa | 18±13µm | Bead |
| 100KGy | 281±26kPa | 16±8µm | Bead |

As a result, as shown in [Table 3], it was confirmed that the ultimate strength and average pore size of the scaffolds prepared with electron beam decreased compared to the scaffolds prepared chemically (Table 1 and Table 2).

### [Example 4]

### Analysis of cell adhesion morphology according to structural differences through scanning electron microscopy

In order to confirm the cell adhesion morphology of two types of scaffolds according to the present invention, human fibroblasts were cultured on the scaffolds for 1, 3 or 7 days, and then imaged using SEM through platinum coating.

As a result, as shown in [FIG. 4], although the same number of cells were injected, it was confirmed that the number of cells adhered to the bead form was different from that to the sponge form. In addition, it was confirmed that in the cell adhesion morphology, the cell end was sharp in the bead form, whereas the cell end was widely spread in the sponge form. This means that the cell adhesion morphology may be controlled according to the preparation method.

### [Example 5]

### Analysis of subcutaneous implantation of scaffolds in rats

In order to confirm the *in vivo* safety and biocompatibility of the bead or sponge-type scaffolds according to the present invention, these were confirmed by histological analysis after performing subcutaneous implantation in rats. As animals, SD Rats (male, 8 weeks old) were used and observed 3 and 6 weeks after subcutaneous implantation.

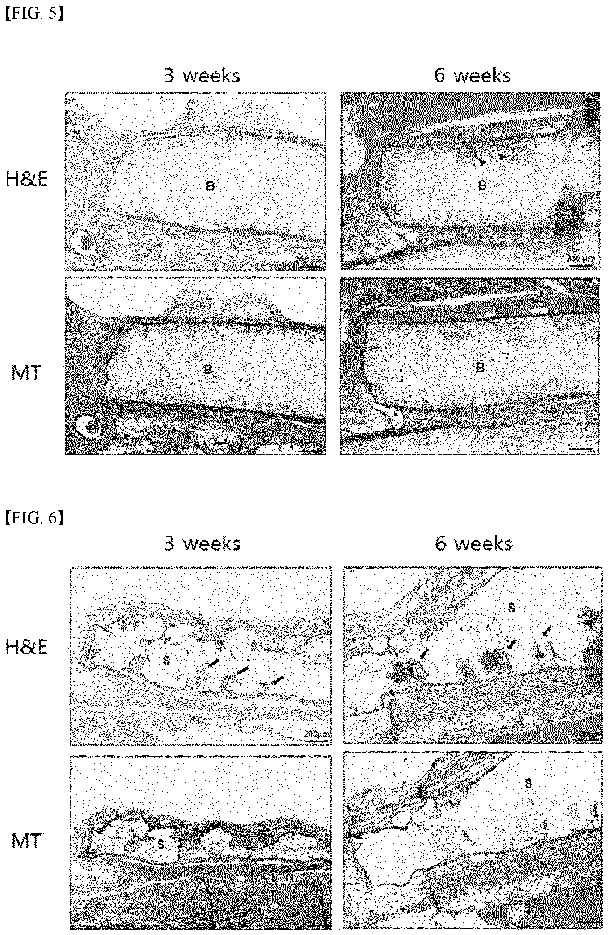

As a result, as shown in [FIG. 5] and [FIG. 6], the form of the scaffold was maintained in the subcutaneous implantation of the bead-type scaffold in the 3-week result, and it was confirmed that cells surrounding the scaffold did not infiltrate therein and formed a fibrous capsule on the surface. In the 6-week result, it was confirmed that the shape of the scaffold was maintained, and it was shown that cell infiltration could be slowed by confirming that some cells were infiltrated inside the scaffold. In addition, it was confirmed from the results of Masson's Trichrome (MT) staining that the infiltrating cells were generating an extracellular matrix. This meant that biocompatibility could be controlled according to the preparation method.

## Claims

1. A method for preparing a scaffold, comprising:
i) preparing a mixed solution by mixing a water-soluble non-degradable acrylic monomer and a water-insoluble non-degradable acrylic monomer with distilled water;
ii) adding a salt and a polar solvent to the mixed solution of step i) to stir; and
iii) cross-linking the water-soluble non-degradable acrylic monomer in the stirred mixed solution of step ii) for polymerizing.

2. The method of claim 1, wherein the water-soluble non-degradable acrylic monomer of step i) is at least one selected from the group consisting of hydroxymethacrylate, hydroxyethylacrylate, ethylacrylate, alkylacrylate, arylacrylate and cyanoacrylate.

3. The method of either claim 1 or claim 2, wherein the water-insoluble non-degradable acrylic monomer of step i) is at least one selected from the group consisting of methylmethacrylate, methylacrylate, alkylmethacrylate, arylmethacrylate and cyanomethacrylate.

4. The method of any one of claims 1 to 3, wherein the water-soluble non-degradable acrylic monomer and the water-insoluble non-degradable acrylic monomer of step i) are mixed at a weight ratio of 50 to 80 : 20 to 50.

5. The method of any one of claims 1 to 4, wherein the cross-linking of step iii) is at least one selected from the group consisting of chemical cross-linking, physical cross-linking, ionic cross-linking and radiation cross-linking.

6. The method of claim 5, wherein an initiator and a catalyst for the chemical cross-linking, for polymerization, are comprised at 1 to 3 wt.% and 0.1 to 1 wt.% based on the total solution weight, respectively.

7. The method of claim 6, wherein the initiator and the catalyst are comprised at a weight ratio of 50 to 80 : 20 to 50.

8. The method of claim 5, wherein the radiation dose for the radiation cross-linking polymerization is 10 kGy to 200 kGy.

9. The method of claim 8, wherein the radiation is at least one selected from the group consisting of gamma rays, electron beam, ion beam and neutron beam.

10. The method of any one of claims 1 to 9, wherein the scaffold is in the form of a bead or sponge.

11. The method of any one of claims 1 to 10, wherein the scaffold is a scaffold for artificial organs.

12. A scaffold prepared by the method of any one of claims 1 to 11.
